# EUROPEAN PATENT SPECIFICATION

(11) **EP 3 472 343 B1**
(45) Date of publication and mention of the grant of the patent: **10.03.2021**
(21) Application number: 17729901.3
(22) Date of filing: 17.06.2017
(51) Int. Cl.: C12Q 1/6827

(54) **NUCLEIC ACID MUTATION DETECTION USING MAGNETIC BEAD ACTUATION AND DETECTION**
NUKLEINSÄUREMUTATIONSDETEKTION MIT MAGNETKUGELAKTUATION UND -DETEKTION
DÉTECTION DE MUTATION D'ACIDE NUCLÉIQUE AU MOYEN D'ACTIONNEMENT DE BILLES MAGNÉTIQUES ET DE LEUR DÉTECTION

(30) Priority: 21.06.2016 EP 16175435
(43) Date of publication of application: 24.04.2019
(73) Proprietor: Koninklijke Philips N.V., 5656 AG Eindhoven (NL)
(72) Inventor: DE REGT, Bram, 5656 AE Eindhoven (NL); FEITSMA, Harma Martine, 5656 AE Eindhoven (NL); EVERS, Toon Hendrik, 5656 AE Eindhoven (NL); VAN ZON, Joannes Baptist Adrianus Dionisius, 5656 AE Eindhoven (NL); HIELTJES, Martinus Johannes, 5656 AE Eindhoven (NL)
(74) Representative: Philips Intellectual Property & Standards
(86) International application number: PCT/EP2017/064854
(87) International publication number: WO 2017/220453

(56) References cited:
- WO-A1-2015/086654
- CN-A- 104 388 570
- US-A1- 2002 119 470
- US-A1- 2007 042 506
- US-A1- 2010 173 290
- US-A1- 2014 134 602
- NGO HOAN T ET AL: "Sensitive DNA detection and SNP discrimination using ultrabright SERS nanorattles and magnetic beads for malaria diagnostics", BIOSENSORS AND BIOELECTRONICS, ELSEVIER BV, NL, vol. 81, 29 January 2016 (2016-01-29), pages 8-14, XP029494716, ISSN: 0956-5663, DOI: 10.1016/J.BIOS.2016.01.073
- BRULS D M ET AL: "Rapid integrated biosensor for multiplexed immunoassays based on actuated magnetic nanoparticles", LAB ON A CHIP: MINIATURISATION FOR CHEMISTRY, PHYSICS, BIOLOGY, MATERIALS SCIENCE AND BIOENGINEERING, ROYAL SOCIETY OF CHEMISTRY, GB, vol. 9, no. 24, 1 January 2009 (2009-01-01), pages 3504-3510, XP002582532, ISSN: 1473-0197, DOI: 10.1039/B913960E [retrieved on 2009-10-15]

## Description

### FIELD OF THE INVENTION

The present invention relates to a method for distinguishing mutations, single nucleotide polymorphisms or other variants in a target nucleic acid sequence from the wild-type sequence in a sample. The present invention further relates to using magnetic bead actuation and/or temperature control.

### BACKGROUND OF THE INVENTION

Kinetic binding experiments are used to determine the association (Kₒₙ) and dissociation (K_{off}) rate constants, assuming that binding follows the law of mass action:

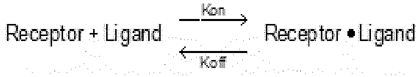

At any given time, the rate at which receptor-ligand complexes form is proportional to the ligand concentration and the number of receptors still unoccupied. The rate of dissociation is proportional to the concentration of receptor-ligand complexes.

Mutations, single nucleotide polymorphism variants (SNVs or SNPs) and other variants in a target nucleic acid sequence are usually best distinguished from the wild-type sequence by discriminating between the respective K_{off}s of any given target nucleic acid containing one or more mutations, SNP variants or other variants and the wild-type nucleic acid in a hybridization assay, in which receptor and ligand are complementary nucleic acids. This is due to there being a bigger difference in K_{off} than in Kₒₙ for mismatched hybridizations.

Discrimination between Kₒₙs is possible in some cases, but requires highly controlled reaction conditions (Knez *et al.,* 2014) that are difficult to provide in point of care settings. Furthermore, an enzymatic reaction in which the enzyme only acts on perfectly matched nucleic acid hybrids is typically added to the Kₒₙ-based reaction to increase specificity (Knez *et al.,* 2014). Integration of enzymes, however, provides additional challenges for integrated systems and each such assay can only determine one expected mutation, SNP variant or other variant or one expected combination of several such mutations, SNP variants or other variants at known positions due to the enzyme requiring a perfectly matched nucleic acid hybrid for activity.

Discrimination between K_{off}s is usually done by performing a hybridization reaction to surface-bound probes and subsequently running a melting curve by gradually increasing the temperature from the hybridization temperature to 90-95°C during which the hybridization signal is monitored (Meuzelaar *et al.,* 2007). Such a two-step process requiring high temperatures is similarly not ideal for point of care settings.

WO 2015/086654 A1, US 2010/173290 A1 and Ngo Hoan T et al.: "Sensitive DNA detection and SNP discrimination using ultrabright SERS nanorattles and magnetic beads for malaria diagnostics", Biosensor and Bioelectronics, Vol. 81, 29 January 2016, pp. 8-14 disclose methods for SNP detection.

### SUMMARY OF THE INVENTION

There is hence a need for improved means and methods for simplified, low-cost, efficient and flexible detection of mutations, SNP variants or other variants in a target nucleic acid sequence and for distinguishing them from the wild-type nucleic acid sequence.

It would furthermore be advantageous if such means and methods could be used in an integrated system in point of care settings, not requiring high temperatures, enzymatic steps or highly controlled reaction conditions.

It would furthermore be advantageous if multiple target nucleic acids containing different mutations, SNP variants, or other variants could be distinguished from each other and the wild-type nucleic acid in the same assay.

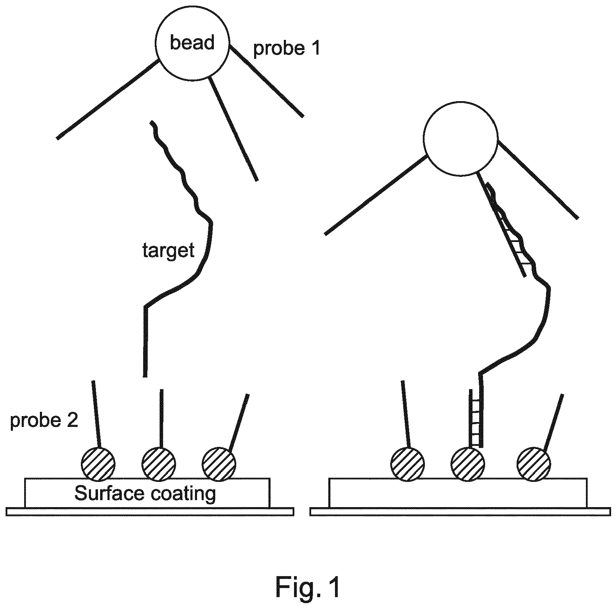

To better address one or more of these concerns, the present invention uses magnetic bead actuation in a hybridization assay with temperature control and an optical readout. The method is based on surface binding of magnetic beads via a sandwich structure with one detection probe bound to a solid surface, and one detection probe bound to a magnetic bead, both of which hybridize with the same target nucleic acid (Fig. 1).

Since both the magnetic actuation and the temperature are controlled in the method of the present invention, mismatches can be detected during the hybridization reaction, eliminating the need for a subsequent melting curve. This not only reduces assay time but also eliminates the need for high temperatures, which in turn reduces issues of evaporation and pressure. Furthermore, no enzymatic reaction is required. These simplifications of the reaction conditions make possible the integration of the method of the present invention into integrated systems. Such an integrated system is, for example, a low-cost microfluidic cartridge. Such cartridges make the present invention more convenient for point of care use.

The present invention also allows for discrimination between multiple target nucleic acids containing different mutations, SNP variants, or other variants and the wild-type nucleic acid in the same assay, since multiple, spatially segregated sets of surface-bound probes (hereinafter surface probes), optionally containing one or more mismatches compared to the probe sequence that is perfectly complementary to the wild-type nucleic acid target can be provided on the same cartridge.

This discrimination between multiple target nucleic acids containing different mutations, SNP variants, or other variants and the wild-type nucleic acid is made more robust by the option to control multiple variants in the method of the present invention. Varying the magnetic stringency of the hybridization at a fixed temperature close to the melting point of the probes leads to differences in the amounts of surface bound magnetic beads depending on the number of mismatches in the hybridization reaction. This embodiment is not part of the invention. Similarly, the magnetic force can be kept constant while the reaction temperature may be varied. This embodiment is not part of the invention either.

In the present invention, combining the variation of both magnetic force and temperature can allow for even greater sensitivity of the assay.

Furthermore, since the present method uses two probes in a sandwich hybridization reaction, more sequence can be covered in one reaction, allowing for fine tuning of assay design and for the detection of mutations, SNP variants, or other variants in two regions of any given target nucleic acid in one reaction.

Finally, addition of a reference gene measurement can be used to determine the presence of a mutation, SNP variant or other variant in a target nucleic acid without requiring a wild-type nucleic acid to compare to.

In a main aspect, the present invention employs a method for detecting mutations or SNP variants or other variants in a target nucleic acid, comprising:
a) providing a bead probe and a surface probe, wherein each probe is an oligonucleotide probe having a sequence complementary to a sequence of the target nucleic acid, optionally containing one or more base changes compared to the sequence of the target nucleic acid, and wherein the bead probe is attached to a magnetic bead, and wherein the surface probe is attached to a solid surface,
b) providing a sample comprising the target nucleic acid, wherein the target nucleic acid may contain one or more mutations and/or variants as compared to the wild-type sequence of the target nucleic acid,
c) contacting the sample with the bead probe and the surface probe,
d) allowing the target nucleic acid to hybridize with the bead probe and the surface probe, forming a sandwich structure, optionally obtaining a first readout of the resulting binding signal
e) applying stringency on the hybridization bond by magnetic force and temperature,
f) determining the amount of magnetic beads remaining in a sandwich structure attached to the surface,
g) repeating steps e) and f) one or more times, and
h) correlating binding signal remaining in a sandwich structure attached to the surface to the presence and/or number of mutations SNP variants or other variants in the target nucleic acid,
   wherein magnetic fields of increasing voltages, and increasing temperatures are applied at the same time or sequentially, wherein said voltages and temperatures are controlled independently of each other

In one embodiment, controlling the temperature comprises increasing the temperature of the sample above the melting temperature of the target nucleic acid containing the mutation or SNP variant or other variant.

In another embodiment, determining the amount of binding signal remaining in a sandwich structure attached to the surface comprises optical detection of the magnetic beads.

In another embodiment, several spatially segregated surface probes are present on the same solid surface and under the same stringency conditions, each detecting a specific variant of the target nucleic acid or a specific region of the target nucleic acid where mutations or SNP variant or other variants may be present. In one embodiment said spatially segregated surface probes comprise a surface probe for the wild-type sequence and one or more surface probes for particular mutations or SNP variants or other variants of interest of the target nucleic acid. In an embodiment, said solid surface is a cartridge.

In another embodiment, the target nucleic acid undergoes an amplification step prior to or during step d).

In another embodiment, the target nucleic acid is derived from a gene involved in drug resistance, optionally from a pathogen, optionally from a viral gene or from a bacterial gene or from a fungal gene. In one embodiment, different strains of a pathogen are identified by detecting variants in the target nucleic acid derived from said pathogenic gene involved in drug resistance. In one embodiment, the bacterial gene involved in drug resistance that the target nucleic acid is derived from is a gene from *Mycobacterium tuberculosis.* In another embodiment, the viral gene is an *Influenza* gene. In an embodiment, the *Influenza* gene can be used for H/N typing.

In another embodiment, the target nucleic acid is derived from a mammalian, preferably human, gene involved in cancer.

In another embodiment, controlling the voltage and temperature reduces the nonspecific binding of nucleic acids with strongly related sequence to the target nucleic acid.

### BRIEF DESCRIPTION OF THE DRAWINGS

Fig 1 shows a schematic representation of surface binding of magnetic beads via a sandwich structure with one detection probe bound to a solid surface, and one detection probe bound to a magnetic bead, both of which hybridize with the same target nucleic acid.
Fig. 2 shows probes (empty boxes), a target wild-type DNA (boxes with hatching leaning right), and the target DNA with mismatches (thick framed boxes with hatching leaning left) introduced on the surface side (top) or bead side (bottom) of the DNA.
Fig. 3 shows the bead count for various probes at increasing temperature and fixed magnetic force for surface side mismatches. Error bars represent standard deviations.
Fig. 4 shows the bead count for various probes at fixed temperature and fixed magnetic force for surface side mismatches. Error bars represent standard deviations.
Fig. 5 shows the bead count for various probes at fixed temperature and increasing magnetic force for surface side mismatches represented as a box plot. N = 2.
Fig. 6 shows the bead count for various probes at increasing temperature and fixed magnetic force for bead side mismatches. N = 2 for all measurements above blank level.

### DETAILED DESCRIPTION OF EMBODIMENTS

Although the present invention will be described with respect to particular embodiments, this description is not to be construed in a limiting sense. The invention is as defined in the appended claims.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20%, preferably ±15%, more preferably ±10%, and even more preferably ±5%.

It is to be understood that the term "comprising" is not limiting. For the purposes of the present invention, the term "consisting of' is considered to be a preferred embodiment of the term "comprising of'. If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims are used to distinguish between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the term "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein below.

The term "nucleic acid" refers to a deoxyribonucleotide or ribonucleotide polymer in either single- or double-stranded form, and also encompasses known analogues of natural nucleotides that can function in a similar manner as naturally occurring nucleotides.

The term "target nucleic acid" refers to a nucleic acid of interest in which the presence or number of mutations, SNP variants, or other variants are to be detected. Bead probes and surface probes are complementary to stretches of the target nucleic acid sequence. The target nucleic acid may optionally be amplified before detection of mutations, SNP variants, or other variants.

The term "mutation" refers to a permanent alteration of the sequence of a nucleic acid. Mutations result from damage to DNA which is not repaired, errors in the process of replication, or from the insertion or deletion of segments of DNA by mobile genetic elements. Mutations can produce discernible changes in the phenotype of an organism. Mutations play a part in both normal and abnormal biological processes including, but not limited to, evolution, cancer, immune system function, and drug resistance.

The term "single nucleotide polymorphism" or "SNP" refers to variation in a single nucleotide that occurs at a specific position in the genome, where each variation is present to some appreciable degree within a population (e.g. >1%). For most SNPs so far discovered, there are only two different bases that occur at that position, but SNPs with three different base variations have been described. SNPs underlie differences in susceptibility to disease; a wide range of human diseases, e.g. sickle-cell anemia, β-thalassemia and cystic fibrosis result from SNPs. The severity of illness and the responsiveness to treatments can also depend on genetic variations. For example, in humans, a single base mutation in the APOE (apolipoprotein E) gene is associated with a higher risk for Alzheimer's disease. The term "variant" refers to any alteration in a nucleic acid that changes its nucleotide sequence.

The terms "oligonucleotide probe" and "probe" refer to a single-stranded nucleic acid sequence that is complementary to at least a portion of the sequence of the target nucleic acid, and, under appropriate conditions, will hybridize with the target nucleic acid. Optionally, the probe sequences contain one or more base changes compared to the target nucleic acid sequence. Typically, such oligonucleotide probes will have a length of about 10 to about 1000 nucleotides, of about 10 to about 800 nucleotides, of about 10 to about 700 nucleotides, of about 10 to about 600 nucleotides, of about 10 to about 500 nucleotides, of about 15 to about 400 nucleotides, of about 15 to about 300 nucleotides, of about 15 to about 200 nucleotides, of about 20 to about 150 nucleotides, of about 20 to about 100 nucleotides, of about 20 to about 90 nucleotides, of about 20 to about 80 nucleotides, of about 20 to about 70 nucleotides, of about 20 to about 60 nucleotides or of about 20 to about 50 nucleotides. Typically, oligonucleotides will have a length of about 20, 30, 40, 50, 60, or 70 nucleotides.

The term "bead probe" refers to an oligonucleotide probe that is immobilized on a magnetic bead.

The term "surface probe" refers to an oligonucleotide probe that is immobilized on a solid surface.

The term "complementary" refers to the sequence of a single-stranded oligonucleotide probe that can base-pair with a strand of the target nucleic acid that has the opposite sense of the probe via hydrogen bonds under appropriate hybridization conditions, thereby forming a double stranded segment of nucleic acid. For the purpose of the present invention, a complementary probe will be understood to be perfectly complementary (also referred to as "matched"), i.e. all of its bases will pair with target nucleic acid bases directly adjacent to each, or to contain base changes to "mismatched" bases in up to half of its positions compared to the perfectly complementary sequence. For example, a twenty nucleotide long probe may contain zero, one, two, three, four, five, six, seven, eight, nine, or ten base changes compared to the perfectly complementary sequence of that probe. The mismatched bases cannot hydrogen bond with the base at the corresponding position in the target nucleic acid. The base changes may be located at adjacent or non-adjacent positions within the probe sequence, or at a mixture of adjacent and non-adjacent positions.

The terms "hybridize" and "hybridization" refer to a phenomenon in which single-stranded nucleic acids anneal to complementary nucleic acids, forming a double-stranded nucleic acid or "hybrid" by forming non-covalent hydrogen bonds between matched purines and pyrimidines. An adenine-thymine or uracil-thymine base pair forms two hydrogen bonds, and a cytosine-guanine base pair forms three hydrogen bonds.

The term "binding signal" refers to the signal resulting from beads bound to the surface via a sandwich structure. The binding signal can be a general signal across the surface, or can be correlated to the specific amount of beads in sandwich structures, depending on the method used for detection of the binding signal.

The terms "sandwich structure" and "sandwich hybridization" refer to a nucleic acid complex in which the target nucleic acid hybridizes with two probes, one being a surface probe and one being a bead probe, at separate, non-overlapping stretches of its nucleic acid sequence, thereby being "sandwiched" between the two probes (Fig. 1). Through this process, a magnetic bead will be bound to the surface that the surface probe is immobilized on.

The term "stringency" for the purpose of the present invention refers to increasingly rigorous conditions that make nucleic acid hybrids more difficult to maintain. Stringency can be applied in the form of magnetic force that acts on magnetic beads bound to the surface via a sandwich structure or in the form of elevated temperatures close to or higher than the melting temperature of the nucleic acid hybrid, or in the form of a combination of magnetic force and elevated temperature, thereby disrupting the non-covalent hydrogen bonds that exist between matched bases. Any of these methods for applying stringency can be combined with a buffer composition that decreases or increases stringency, i.e. facilitates or makes harder the maintenance of nucleic acid hybrids. This allows for tight control of stringency levels.

The term "melting temperature" refers to the temperature that leads to 50% disruption of nucleic acid hydrogen-bond base pairing in a double-stranded hybrid, i.e. at which 50% of the hybrid are converted into single-stranded nucleic acids. This process is also called "denaturation". The stability of the nucleic acid hybrid depends partially on its sequence, since guanine-cytosine pairs form one more hydrogen bond than uracil-adenine or thymine-adenine pairs, therefore requiring more energy, i.e. a higher melting temperature, for the disruption of the additional hydrogen bond. The length of the hybrid also plays a role in determining its melting temperature, since additional energy is required for each base pair. Furthermore, the number and location of mismatches will also influence the melting temperature, with any mismatches lowering the melting temperature. Mismatches at central positions within the hybrid will lower the melting temperature further than mismatches at terminal positions. The shorter the stretches of adjacent matched bases become, the lower the melting temperature will be. The melting temperature is also dependent on ionic strength and the concentration of DNA. In the sandwich structure of the present invention, magnetic forces that put additional strain on the hydrogen-bonds of the hybrid lower the melting temperature of the hybrid.

The term "spatially segregated" refers to surface probes immobilized to isolated spots on a surface, i.e. areas that contain only one species of surface probe separated by space in which no surface probe is attached to the surface. For example, a microfluidic cartridge might contain four separate spots, each of which has a different species of surface attached to the cartridge surface.

The term "wild-type" refers to the naturally occurring sequence of a nucleic acid or gene. If multiple alleles of a nucleic acid or gene exist, the one most predominant in a population is considered the wild-type allele.

The term "amplification" refers to the generation of additional copies of the target nucleic acid or stretches thereof, using e.g. polymerase chain reaction (PCR) or any other method suitable for the amplification of nucleic acids.

The term "drug resistance" refers to the reduction in effectiveness of a drug in curing a disease or condition or in killing or inhibiting a pathogen. Such drug resistance can be conferred by a multitude of processes, for example by the acquisition of mutations, SNP variants, or other variants in nucleic acid sequences in a host, and/or the expression of the gene product of the altered nucleic acid sequence. When an organism or cell is resistant to more than one drug, it is said to be multidrug-resistant.

The term "gene involved in drug resistance" refers to any gene that contributes to the drug resistance of an organism, such as a pathogen or cell. The main mechanisms by which resistance to a drug is exhibited are (1) drug inactivation or modification, e.g. enzymatic deactivation or degradation, (2) alteration of the drug target, e.g. base changes in nucleic acid sequences or amino acid changes in protein or peptide sequences that a drug binds to or that lead to conformational changes that make the binding site inaccessible to the drug or lower its affinity for binding the drug, (3) alteration of the metabolic pathway the drug inhibits, e.g. use of alternative precursors, use of redundant enzymes that can also facilitate the step blocked by the drug, or uptake and use of the pathway product or intermediate product from the surroundings, (4) reduced drug accumulation, e.g. by decreasing drug permeability and/or increasing active efflux (pumping out) of the drugs across the cell surface, (5) overexpression of a protein target of a drug, e.g. by increased transcription and translation, or by acquisition of additional copies of the gene encoding the protein target, and (6) acquisition of multiple copies of a nucleic acid target of a drug.

The term "pathogen" refers to an infectious agent such as, for example, but not limited to, a virus, bacterium, prion, fungus, viroid, or parasite that causes disease in its host. The host may be a human, an animal, a plant, a fungus, or a microorganism, including other pathogens.

The term "strains of a pathogen" refers to a genetically distinct subtype of a species of pathogen.

The term "gene involved in cancer" refers to any gene that contributes to the predisposition for and/or development of and/or progression of cancer. Such genes include "oncogenes", which have the potential to cause cancer, and "tumor suppressor genes", which protect a cell from one step on the path to cancer. Oncogenes comprise growth factors that induce cell proliferation, receptor tyrosine kinases that transduce signals for cell growth and differentiation, cytoplasmic tyrosine kinases that mediate the responses to and the activation receptors of cell proliferation, differentiation, cell survival and apoptosis, cytoplasmic serine/threonine kinases and their regulatory subunits that are involved in organism development cell cycle regulation, cell proliferation, differentiation and survival and apoptosis, regulatory GTPases that are involved in signaling a major pathway leading to cell proliferation, and transcription factors that regulate the transcription of genes that induce cell proliferation or of other oncogenes. Typically, oncogenes need to be overexpressed or mutated compared to the wild-type gene to cause cancer. Tumor suppressor genes comprise genes that are involved in repression of genes that are essential for cell cycle progression, coupling the cell cycle to DNA damage, i.e. inhibiting the cell cycle in the presence of damaged DNA, initiation of apoptosis, cell adhesion, contact inhibition and DNA repair. Tumor suppressor genes must typically be mutated, underexpressed or silenced for cancer to develop, although, as opposed to oncogenes, both alleles of the tumor suppressor gene must be so affected.

The term "nonspecific binding" relates to the hybridization of non-complementary single-stranded nucleic acids. Such nonspecific binding is typically weak, since fewer hydrogen bonds are formed, and can be reduced or eliminated by applying stringency in the form of a magnetic force or voltage, elevated temperatures above the melting temperature of the nonspecific hybrid but below the melting temperature of the specific hybrid, or a combination of the two.

The term "strongly related" relates to a sequence of a nucleic acid that is similar but not identical to the target nucleic acid. The similarity is lower than the similarity of the probe with the highest number of mismatched bases.

In a main aspect, the present disclosure employs a method for detecting mutations or SNP variants or other variants in a target nucleic acid, comprising: a) providing a bead probe and a surface probe, wherein each probe is an oligonucleotide probe having a sequence complementary to a stretch of the sequence of the target nucleic acid, optionally containing one or more base changes compared to the sequence of the target nucleic acid, and wherein the bead probe is attached to a magnetic bead, and wherein the surface probe is attached to a solid surface, b) providing a sample comprising the target nucleic acid, wherein the target nucleic acid may contain one or more mutations and/or variants as compared to the wild-type sequence of the target nucleic acid, c) contacting the sample with the bead probe and the surface probe, d) allowing the target nucleic acid to hybridize with the bead probe and the surface probe, forming a sandwich structure, optionally obtaining a first readout of the binding signal attached to the surface e) applying stringency on the hybridization bond, optionally by magnetic force and/or temperature, f) determining the binding signal remaining in a sandwich structure attached to the surface, g) optionally repeating steps e) and f) one or more times, and h) correlating the binding signal remaining in a sandwich structure attached to the surface to the presence and/or number of mutations SNP variants or other variants in the target nucleic acid.

The target nucleic acid may, for example, be DNA, RNA or cDNA. The target nucleic may contain one or more mutations compared to the wild type sequence of the target nucleic acid. The target nucleic acid may contain one or more SNPs compared to the predominant sequence of the target nucleic acid that is considered to be its wild type. The target nucleic acid may contain one or more other variants in its sequence compared to the wild type sequence of the target nucleic acid. Furthermore, the target nucleic may contain one or more mutations and one or more SNPs compared to the wild type sequence. The target nucleic acid may also contain one or more mutations and one or more other variants compared to the wild type sequence. The target nucleic acid may contain one or more SNPs and one or more other variants compared to the wild type sequence. Finally, the target nucleic acid may also contain one or more mutations, one or more SNPs and one or more other variants compared to the wild type sequence of the target nucleic acid.

Many important molecular applications, such as DNA oligonucleotide arrays, utilize synthetic oligonucleotides attached to solid supports. For this attachment to take place, the oligonucleotides must be modified with a functional group in order to have attachment to a reactive group on a solid surface. The oligonucleotides can be attached to flat two-dimensional surfaces, such as glass slides or polymer surfaces, as well as to three-dimensional surfaces such as micro-beads and micro-spheres. In the present invention, the bead probe is immobilized onto a magnetic bead and the surface probe is immobilized onto a solid surface. The most accessible approach for this is to synthesize individual oligonucleotides and subsequently immobilize them to a solid surface or bead. The more commonly used chemistries available for solid support attachment include: a) hydrazide-modified oligonucleotides covalently linked to aldehyde or epoxide treated surfaces, b) amine-modified oligos covalently linked to an activated carboxylate group or succinimidyl ester, c) succinylated oligonucleotides linked to aminophenyl or aminopropyl-derivatized glass surfaces, d) disulfide modified olignocleotides linked to a mercaptosilanized glass surface, e) thiol-modified oligos covalently linked via an alkylating reagent such as an iodoacetamide or maleimide, f) digoxigenin NHS ester, g) cholesterol-TEG, and h) biotin-modified oligos captured by immobilized streptavidin. The bead probe and surface probe may also be attached via other chemistries that the person skilled in the art will be aware of.

The bead probe and surface probe being each complementary to stretches of the target nucleic acid may be complementary to terminal or internal stretches of the target nucleic acid sequence. The bead probe and surface probe may be complementary to opposite terminal regions of the target nucleic acid, i.e. the bead probe may be complementary to one end, and the surface probe to the other end. The bead probe may be complementary to one end of the target nucleic acid, and the surface probe may be complementary to a stretch of target nucleic acid located away from the ends of the target nucleic acid. The bead probe may be complementary to one end of the target nucleic acid, and the surface probe may be complementary to a stretch of target nucleic acid located near the sequence to which the bead probe is complementary. The surface probe may be complementary to one end of the target nucleic acid, and the bead probe may be complementary to a stretch of target nucleic acid located away from the ends of the target nucleic acid. The surface probe may be complementary to one end of the target nucleic acid, and the bead probe may be complementary to a stretch of target nucleic acid located near the sequence to which the surface probe is complementary. The surface probe and the bead probe may both be complementary to internal stretches of the target nucleic acid sequence. The surface probe and the bead probe may both be complementary to internal stretches of the target nucleic acid sequence at locations near each other. They may never be complementary to the same stretch of target nucleic acid sequence or to each other.

Contacting the sample comprising the target nucleic acid with the surface probe is done by bringing into contact the surface on which the surface probe is immobilized and the sample. This may occur by adding the sample to a slide, array, cartridge, well or tube. Contacting the sample comprising the target nucleic acid with the bead probe is done by bringing into contact the sample and magnetic beads on which the bead probe is immobilized. This may occur by adding beads to the sample or the sample to beads on a slide, array, cartridge, or in a well or tube. It is preferred that these steps be carried out simultaneously, i.e. by adding the sample to a surface of a well, tube, chamber or other compartment which also contains the magnetic beads. Most preferred is the addition of the sample to a microfluidic cartridge compartment, the surface of which has the surface probe immobilized to it and which contains magnetic beads with the bead probe mobilized on their surface. An example of such a microfluidic cartridge is one described in US 2010/310423 A1, also referred to as a Magnotech™ cartridges. Magnotech™ cartridges are disposable cartridges that fill themselves with a single drop of liquid and subsequently require no further fluid movement. The entire assay process within these cartridges is performed by external application of magnetic fields to control the movement of magnetic nanoparticles, with which the cartridge comes preloaded, within the cartridge. These fields are applied by small electromagnets situated beneath and above the cartridge.

Hybridization occurs once the sample has been contacted with both the surface probe and the bead probe. The optimal temperature, buffers and other reaction conditions will depend on a number of factors known to the person skilled in the art. Of crucial importance for the determination of optimal temperature and conditions is the length of the oligonucleotide probes, the content of guanine and cytosine, as well as the maximally tolerated number of mismatches between the two sequences, which factor heavily into the melting temperature for any given nucleic acid hybrid. The hybridization reaction temperature will be below the melting temperature to permit the formation of hydrogen bonds. An exemplary protocol for hybridization of target nucleic acid with a bead probe and a surface probe is as follows for bead and surface probes with a length of 20 nucleotides and 40-55% guanine + cytosine consits of an initial step in which the washing magnet is turned on. This enables a reference measurement in which any artefacts already present on the surface can be detected. This is followed by a short collection step using the horseshoe magnet in which beads are concentrated over the spot containing the surface probes. A longer binding step using the horseshoe magnet follows the collection step, allowing beads to bind to the surface via the target containing potential mismatches.

Following the formation of sandwich structures consisting of one surface probe, one target nucleic acid, and one bead probe, effectively linking the magnetic bead to the surface, stringency is applied to the hybrid structures in a first, short washing step, after which another collection and binding step are carried out, and a second, longer washing step to assess the strength of the binding between the beads and the surface. In one embodiment, applying stringency to the hybridization bond comprises applying magnetic fields of increasing voltages to the sample. The person skilled in the art will be aware that the range of the magnetic field required to lead to the melting of a hybrid depends on the length of the probes, i.e. the length of the hybridized sequence, the content of guanine and cytosine residues, and the number of mismatches within the nucleic acid hybrid. An exemplary range of magnetic field strength to increase stringency on the hybridization of target nucleic acid with a bead probe and a surface probe for bead and surface probes with a length of 20 nucleotides, 50-55% guanine + cytosine at 54°C, and zero, one or two mismatches, a temperature close to the melting temperature of a hybrid of the target nucleic acid with perfectly matched probes, is from 0 to IV, more preferably from 0.2 to IV, most preferably from 0.4 to 0.8V in 0.1V increments lasting 30 seconds each (see Example 4). Other variables such as frequency and duty cycle of the magnets are not altered during this period.

In another embodiment, stringency is applied by increasing the temperature of the sample above the melting temperature of the target nucleic acid containing the mutation or SNP variant or other variant. An exemplary range of temperatures to increase stringency on the hybridization of target nucleic acid with a bead probe and a surface probe for bead and surface probes with a length of 20 nucleotides, 40-55% guanine + cytosine at 54°C and between 0 and 10 mismatches is from 45 to 60°C, more preferably from 48 to 58°C, most preferably from 51 to 57°C (see Example 2).

In another embodiment, stringency is applied by keeping the sample at a constant temperature close to the melting point of the probes, preferably above the melting temperature of the target nucleic acid containing the mutation or SNP variant or other variant. An exemplary constant temperature to increase stringency on the hybridization of target nucleic acid with a bead probe and a surface probe for bead and surface probes with a length of 20 nucleotides, 50-55% guanine + cytosine and between 0 and 2 mismatches is at 57°C (see Example 3).

In another embodiment, increasing voltages and increasing temperatures are applied at the same time or sequentially, wherein said voltages and temperatures are controlled independently of each other. An exemplary range of magnetic field strength to increase stringency on the hybridization of target nucleic acid with a bead probe and a surface probe for bead and surface probes with a length of 20 nucleotides, 40-55% guanine + cytosine at 54°C, and between zero and ten mismatches is from 0 to IV, more preferably from 0.2 to IV, most preferably from 0.4 to 0.8V in 0.1V increments lasting 30 seconds each. Other variables such as frequency and duty cycle of the magnets are not altered during this period. An exemplary range of temperatures is from 45 to 60°C, more preferably from 48 to 58°C, most preferably from 51 to 57°C.

In an embodiment, controlling the voltage and/or temperature also has the effect of reducing the nonspecific binding of nucleic acids with strongly related sequence to the target nucleic acid, since non-specific hybrids will contain a greater number of mismatches than specific ones with a low number of mutations, SNPs or other variants in the target nucleic acid, and therefore have a lower melting temperature and fewer bonds to be strained by magnetic force pulling apart the nucleic acid strands.

After stringency has been applied to the hybridization, the binding signal still bound to the surface via a sandwich structure is determined. Similarly, an optional first-read out of the binding signal can be performed after the hybridization step before stringency is applied. In an embodiment, determining the binding signal remaining in a sandwich structure attached to the surface comprises optical detection of the magnetic beads. Optical detection may occur by any method known in the art, for example by counting by eye beads in light, phase contrast, or electron microscopic images, or by Frustrated Total Internal Reflection (FTIR), which can be used with a convenient, low-cost microfluidic cartridge, such as the Magnotech™ cartridge. In FTIR, light is coupled into the sample at an angle of total internal reflection. If no beads are present close to the sample surface, the light is completely reflected. If, however, magnetic beads are bound to the surface, the condition of total internal reflection is violated, a portion of the light is scattered into the sample and thus the amount of light reflected by the surface is decreased. By measuring the intensity of the reflected light with an optical detector, it is possible to estimate the amount of magnetic beads bound to the surface. This allows for an estimate of the amount of the specific molecules of interest present within the sample.

Optionally, the steps of applying stringency and determining the binding signal bound to the surface are repeated several times, for example once, twice, three times, four times, five times, six times, seven times, eight times, nine times, ten times, and so on, up to one thousand times. Each repetition should lead to a more precise determination of variants in a target nucleic acid, since non-specific or weak binding will be eliminated more thoroughly. Furthermore, variants with very similar hybridization energy may be told apart by comparing the number of beads remaining bound to the surface over several repetitions.

Once the binding signal bound to the surface is determined, it is correlated to the presence and/or number of mutations, SNP variants or other variants in the target nucleic acid, since fewer mismatches between either the surface probe or the bead probe and the target nucleic acid will lead to a higher number of beads still bound to the surface under stringent conditions. If a range of stringency is applied, perfectly complementary sequences will retain beads on the surface to a point of higher stringency than complementary sequences containing mismatches. Optionally, the stringency conditions at which no more or reduced numbers of beads are bound to the surface can be compared to a wild type sequence or reference sequence.

In one embodiment, several spatially segregated surface probes are present on the same solid surface and under the same stringency conditions, each detecting a specific variant of the target nucleic acid or a specific region of the target nucleic acid where mutations or SNP variant or other variants may be present. Spatial segregation can be achieved for example in the form of separate droplets, wells, slots, indents, chambers or printed grids or by other means known to the person skilled in the art on slides, arrays, plates, cartridges, chips or other surfaces know to the skilled person. In a preferred embodiment, the solid surface to which the several spatially segregated probes are attached is a cartridge. In a more preferred embodiment, the cartridge is a microfluidic cartridge. In an especially preferred embodiment, the microfluidic cartridge is a Magnotech™ cartridge.

In another embodiment, the spatially segregated surface probes comprise a surface probe for the wild-type sequence and one or more surface probes for particular mutations or SNP variants or other variants of interest of the target nucleic acid. The wild type sequence may serve as a reference for the stringency conditions required to melt the nucleic acid hybrid. The mutations or SNP variants or other variants of interest may be those known to cause disease or predisposition to disease or to cause drug resistance or drug tolerance, or any other feature of interest that the person known in the art can envision.

In an embodiment, the target nucleic acid undergoes an amplification step prior to or during step d) in which additional copies of the target nucleic acid or stretches thereof are generated using e.g. polymerase chain reaction (PCR), loop mediated isothermal amplification, nucleic acid sequence based amplification, strand displacement amplification, multiple displacement amplification, or any other method suitable for the amplification of nucleic acids. The person skilled in the art will be able to envision the method best suited for the particular application of the present invention.

In an embodiment, the target nucleic acid is derived from a gene involved in drug resistance, optionally from a pathogen, optionally from a viral gene or from a bacterial gene or from a fungal gene. Such pathogens comprise but are not limited to, for example, methicillin-resistant *Staphylococcus aureus* (MRSA), multi-drug resistant bacteria, Influenza Virus, Respiratory Syncytial Virus (RSV), Hepatitis C Virus (HCV), Human Immunodeficiency Virus (HIV), Human Papillomavirus (HPC), Group B *Streptococcus, Chlostridium difficile.* The genes involved in drug resistance are typically those that a drug binds to. Mutations can lead to alteration of the binding of the drug to the drug target, e.g. base changes in nucleic acid sequences or amino acid changes in protein or peptide sequences that a drug binds to decrease the chemical interactions between drug and drug target or lead to conformational changes that make the binding site inaccessible to the drug or lower its affinity for binding the drug. To determine the presence of any mutations in such a gene, probes that are perfectly complementary to the wild type sequence covering the whole gene or region of nucleic acid are printed spatially separated on the same surface. If any of the probes show a lesser degree of retaining beads bound to the surface under stringent conditions, this indicates the presence of a mismatch, and therefore a mutation in the part of the gene or region that is covered by that particular probe. Advantageously, this will detect the presence of any possible mutation and is not limited to a design for a specific mutation that is known in the art.

In another embodiment, different strains of a pathogen are identified by detecting variants in the target nucleic acid derived from said pathogenic gene involved in drug resistance. Such pathogens and genes include but are not limited to, for example, Influenza virus, HPV, HIV, HCV, *Plasmodium, C. difficile* and their genes. The distinction of targets with variants may happen at the end of the protocol, wherein at first, at a relatively low level of stringency, all strains of a pathogen will bind to a probe that is perfectly complementary to the sequence from a particular strain of interest or reference, determining the presence of a certain pathogen in a sample from which the target nucleic acid containing sample was obtained, and wherein upon further increase of the level of stringency, variants will be detected by their differential binding to the probe due to mismatches in the nucleic acid hybrids, determining the presence of new or known strains of the pathogen with base variants compared to the strain of interest or reference. This is relevant for example for detection of pathogens with highly varying strains such as *Influenza* and *Plasmodium.*

In one embodiment, the bacterial gene involved in drug resistance that the target nucleic acid is derived from is a gene from *Mycobacterium tuberculosis.* Such genes include but are not limited to, for example, genes involved in resistance to rifampicin, isoniazid, ethionamide, isoniazid-ethionamide, streptomycin, fluoroquinolones, pyrazinamide, ethambutol.

In another embodiment, the target nucleic acid is derived from a mammalian gene involved in cancer. In a preferred embodiment, the mammalian gene is a human gene. Such a gene may be an oncogene or a tumor suppressor gene. For example, such a gene might be a growth factor, a receptor tyrosine kinase, a cytoplasmic tyrosine kinase, a cytoplasmic serine/threonine kinase, a regulatory GTPase, gene involved in repression of cell cycle progression genes, or a gene coupling the cell cycle to DNA damage. Probes exactly matching the target nucleic acid wild type and a particular target nucleic acid variant known to be involved in cancer may be printed spatially separated on the same surface. If the variant-probe retains a higher amount of beads bound to the surface than the wild type probe under stringent conditions, the variant is present. This is especially relevant for detection of mutations genes in cancer, where the presence of certain specific oncogenic variants such as in KRAS or BRAF influences the choice of therapy.

### EXAMPLES

The methods of the invention for detecting mutations, SNP variants, or other variants in a target nucleic acid are supported and illustrated by reference to the following examples. It has to be emphasized that these examples should by no means be construed as limiting the scope of the invention, which is as defined in the appended claims.

### 1. Probe sequence design

By designing a probe-target combination with an increasing number of mismatched base pairs in the hybridizing part of the sequence a difference between said sequences could be detected. Ideally, all mismatched sequences should yield a lower bead count than the completely matching probe. In practice it is fairly unlikely that the difference between the matching probe and the probe with one mismatch on the edge will be detectable. Therefore, probes used to detect mismatches should be designed in such a way that the mismatch appears somewhere near the middle of the probe.

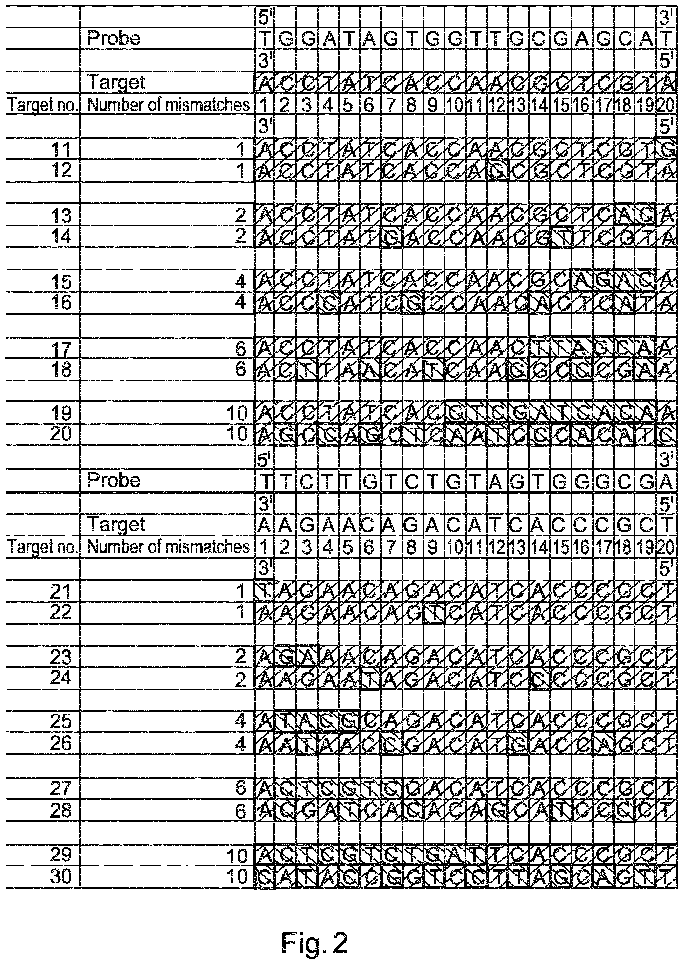

Because each assay contains two probes, one bead probe and one surface probe, mismatches have been introduced on both sides of the target (Fig. 2). The SEQ IDs for all sequences are listed in the table below:

| **Sequence name** | **SEQ ID No.** |
|---|---|
| Surface probe (perfectly complementary) | 1 |
| Target nucleic acid surface side wild-type (Reference) | 2 |
| Target 11 | 3 |
| Target 12 | 4 |
| Target 13 | 5 |
| Target 14 | 6 |
| Target 15 | 7 |
| Target 16 | 8 |
| Target 17 | 9 |
| Target 18 | 10 |
| Target 19 | 11 |
| Target 20 | 12 |
| Bead probe (perfectly complementary) | 13 |
| Target nucleic acid bead side wild-type (Reference) | 14 |
| Target 21 | 15 |
| Target 22 | 16 |
| Target 23 | 17 |
| Target 24 | 18 |
| Target 25 | 19 |
| Target 26 | 20 |
| Target 27 | 21 |
| Target 28 | 22 |
| Target 29 | 23 |
| Target 30 | 24 |

### 2. Applying stringency by varying the temperature at fixed magnetic force on mismatches in the surface probe

DNA targets with an increasing amount of mismatches in the sequence binding to the surface probe were hybridized to the surface and with beads that carry bead probes in microfluidic cartridges with a fixed actuation protocol at 45 °C. The bound beads were then washed away by activating the wash magnet at 0.4 V at increasing temperatures. The actuation protocol used consists of an initial step in which the washing magnet is turned on. This enables a reference measurement in which any artefacts already present on the surface can be detected. This is followed by a short collection step using the horseshoe magnet in which beads are concentrated over the spot containing the surface probes. A longer binding step using the horseshoe magnet follows the collection step, allowing beads to bind to the surface via the target containing potential mismatches. A short washing step follows, after which another collection and binding step are carried out. Finally, a longer washing step is done to assess the strength of the binding between the beads and the surface, during which the temperature is increased in one degree Celsius increments lasting 30 seconds each up to 60°C.

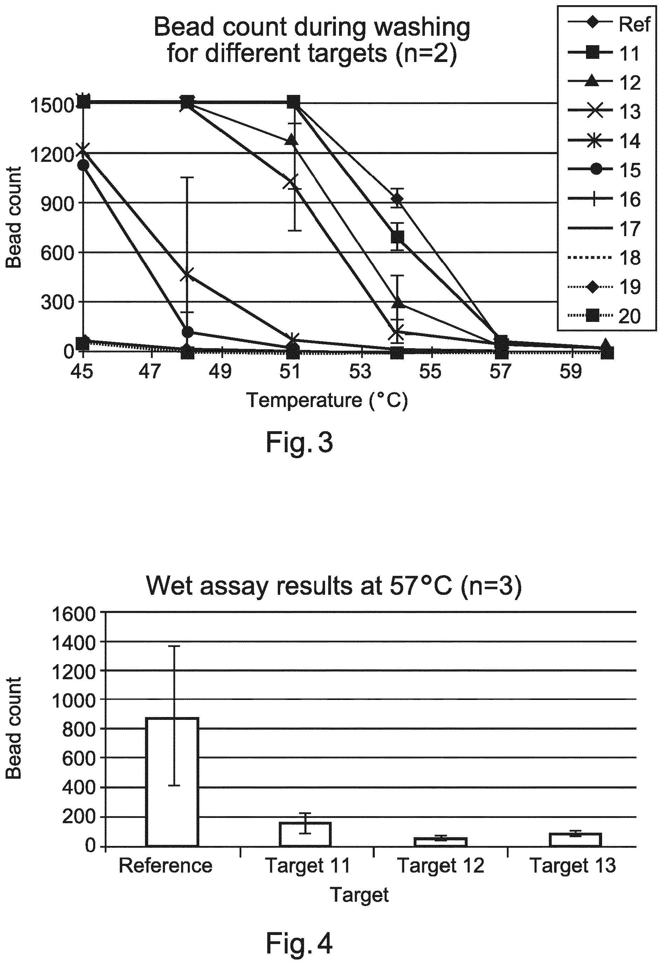

All targets with mismatches show a bead count at 54 °C below the reference. Targets with one (target 11 & 12) or two (13 and 14) mismatches still show a bead count at 54 °C. All bead counts for targets containing one or more mismatches are significantly different from the wild-type target bead count at this temperature (Fig. 3).

### 3. Applying stringency at fixed temperature at fixed magnetic force on mismatches in the surface probe

DNA targets with an increasing amount of mismatches in the sequence binding to the surface probe were hybridized spatially segregated from each other to the surface and with beads that carry bead probes in the same microfluidic cartridges with a fixed actuation protocol at 45 °C. The bound beads were then washed away by actuation (Fig. 4). The actuation protocol used consists of an initial step in which the washing magnet is turned on at 0.4V. This enables a reference measurement in which any artefacts already present on the surface can be detected. This is followed by a short collection step using the horseshoe magnet in which beads are concentrated over the spot containing the surface probes. A longer binding step using the horseshoe magnet follows the collection step, allowing beads to bind to the surface via the target containing potential mismatches. A short washing step follows, after which another collection and binding step are carried out. Finally, a longer washing step is done to assess the strength of the binding between the beads and the surface them at a fixed temperature of 57 °C.

A significant difference in bead count is obtained between each assayed target containing one or more mismatches and the wild-type target, making it possible to distinguish between a perfectly complementary target (*Reference*) and a target with one *(target 11 and 12)* or more (target 13) mismatches.

### 4. Applying stringency by varying magnetic force at fixed temperature on mismatches in the surface probe

DNA targets with an increasing amount of mismatches in the sequence binding to the surface probe were hybridized to the surface and with beads that carry bead probes in microfluidic cartridges with a fixed actuation protocol at 45 °C. The actuation protocol used consists of an initial step in which the washing magnet is turned on. This enables a reference measurement in which any artefacts already present on the surface can be detected. This is followed by a short collection step using the horseshoe magnet in which beads are concentrated over the spot containing the surface probes. A longer binding step using the horseshoe magnet follows the collection step, allowing beads to bind to the surface via the target containing potential mismatches. A short washing step follows, after which another collection and binding step are carried out. Finally, a longer washing step is done to assess the strength of the binding between the beads and the surface. The voltage of the washing magnet is increased from 0.4 to 0.6V to 0.8V in 0.2V increments lasting 30 seconds each while keeping the reaction temperature fixed at 54 °C. Other variables such as frequency and duty cycle of the magnets are not altered during this period.

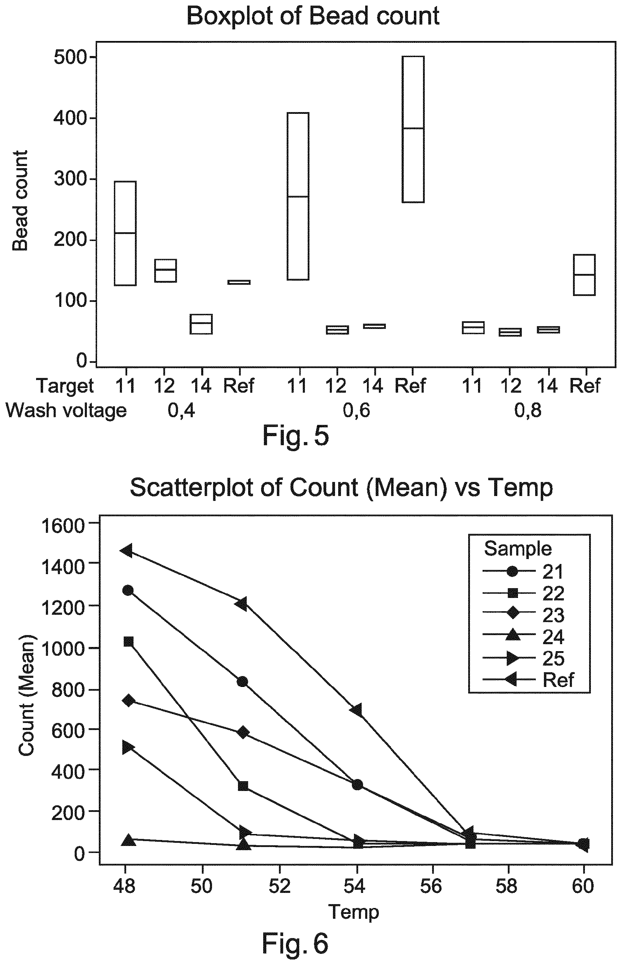

The measured bead count decreased for all measured targets, but it decreased more for targets with a higher number of mismatches, and more for those targets with mismatches in the center of the hybridized sequence (Fig. 5).

### 5. Applying stringency by varying the temperature at fixed magnetic force on mismatches in the bead probe

DNA targets with an increasing amount of mismatches in the sequence binding to the bead probe were hybridized to the surface via a surface probe and with beads in microfluidic cartridges with a fixed actuation protocol at 45 °C. The bound beads were then washed away by activating the wash magnet at 0.4V at increasing temperatures. The actuation protocol used consists of an initial step in which the washing magnet is turned on. This enables a reference measurement in which any artefacts already present on the surface can be detected. This is followed by a short collection step using the horseshoe magnet in which beads are concentrated over the spot containing the surface probes. A longer binding step using the horseshoe magnet follows the collection step, allowing beads to bind to the surface via the target containing potential mismatches. A short washing step follows, after which another collection and binding step are carried out. Finally, a longer washing step is done to assess the strength of the binding between the beads and the surface, during which the temperature is increased in one degree Celsius increments lasting 30 seconds each up to 60°C.

Introducing the mismatches to the side of the target nucleic acid binding to the bead probe gives very similar results to introducing mismatches to the side of the target nucleic acid binding the surface probe, indicating that mismatches can be detected on both sides of the target (Fig. 6).

### LIST OF REFERENCE SIGNS:

1. Knez K., Spasic D., Janssen K.P.F. and Lammertyn J. (2014) Emerging technologies for hybridization based single nucleotide polymorphism detection. Analyst 139: 353-370
2. Meuzelaar L.S., Hopkins K., Liebana E. and Brookes A.J. (2007) DNA Diagnostics by surface-bound melt-curve reactions. J Mol Diagn 9(1): 30-41
3. US 2010/310423 A1

## Claims

1. A method for detecting mutations or SNP variants or other variants in a target nucleic acid, comprising:
a) providing a bead probe and a surface probe, wherein each probe is an oligonucleotide probe having a sequence complementary to a sequence of the target nucleic acid, optionally containing one or more base changes compared to the sequence of the target nucleic acid, and wherein the bead probe is attached to a magnetic bead, and wherein the surface probe is attached to a solid surface,
b) providing a sample comprising the target nucleic acid, wherein the target nucleic acid may contain one or more mutations and/or variants as compared to the wild-type sequence of the target nucleic acid,
c) contacting the sample with the bead probe and the surface probe,
d) allowing the target nucleic acid to hybridize with the bead probe and the surface probe, forming a sandwich structure, optionally obtaining a first readout of the binding signal attached to the surface,
e) applying stringency on the hybridization bond by magnetic force and temperature,
f) determining the binding signal remaining in a sandwich structure attached to the surface,
g) repeating steps e) and f) one or more times, and
h) correlating the binding signal remaining in a sandwich structure attached to the surface to the presence and/or number of mutations, SNP variants, or other variants in the target nucleic acid,
wherein magnetic fields of increasing voltages, and increasing temperatures are applied at the same time or sequentially and wherein said voltages and temperatures are controlled independently of each other.

2. Method according to claim 1, wherein controlling the temperature comprises increasing the temperature of the sample above the melting temperature of the target nucleic acid containing the mutation, or SNP variant or other variant.

3. Method according to any one of the preceding claims, wherein determining the binding signal remaining in a sandwich structure attached to the surface comprises optical detection of the magnetic beads.

4. Method according to any one of the preceding claims, wherein several spatially segregated surface probes are present on the same solid surface and under the same stringency conditions, each detecting a specific variant of the target nucleic acid or a specific region of the target nucleic acid where mutations or SNP variant or other variants may be present.

5. Method according to claim 4, wherein said spatially segregated surface probes comprise a surface probe for the wild-type sequence and one or more surface probes for particular mutations or SNP variants or other variants of interest of the target nucleic acid.

6. Method according to claim 4 or 5, wherein the solid surface is a cartridge.

7. Method according to any one of the preceding claims, wherein the target nucleic acid undergoes an amplification step prior to or during step d).

8. Method according to any one of the preceding claims, wherein the target nucleic acid is derived from a gene involved in drug resistance, optionally from a pathogen, optionally from a viral gene or from a bacterial gene or from a fungal gene.

9. Method according to claim 8, wherein different strains of a pathogen are identified by detecting variants in the target nucleic acid.

10. Method according to claim 8 or 9, wherein the bacterial gene is a gene from *Mycobacterium tuberculosis.*

11. Method according to any one of claims 1 to 7, wherein the target nucleic acid is derived from a mammalian, preferably human, gene involved in cancer.

12. Method according to any one of the preceding claims, wherein controlling the voltage and temperature reduces the nonspecific binding of nucleic acids with strongly related sequence to the target nucleic acid.

## Patentansprüche

1. Verfahren zur Detektion von Mutationen oder SNP-Varianten oder anderen Varianten in einer Zielnukleinsäure, umfassend:
a) Bereitstellen einer Kügelchensonde und einer Oberflächensonde, wobei jede Sonde eine Oligonukleotidsonde mit einer Sequenz ist, die zu einer Sequenz der Zielnukleinsäure komplementär ist, und gegebenenfalls eine oder mehrere Basenänderungen im Vergleich zur Sequenz der Zielnukleinsäure enthält, und wobei die Kügelchensonde an einem magnetischen Kügelchen angebracht ist, und wobei die Oberflächensonde an einer festen Oberfläche angebracht ist,
b) Bereitstellen einer Probe, die die Zielnukleinsäure umfasst, wobei die Zielnukleinsäure eine oder mehrere Mutationen und/oder Varianten im Vergleich zur Wildtypsequenz der Zielnukleinsäure enthalten kann,
c) Kontaktierung der Probe mit der Kügelchensonde und der Oberflächensonde,
d) Ermöglichen, dass die Zielnukleinsäure mit der Kügelchensonde und der Oberflächensonde hybridisiert, wobei eine Sandwichstruktur gebildet wird, wobei gegebenenfalls ein erstes Auslesen des an die Oberfläche gebundenen Bindungssignals erhalten wird;
e) Anwenden von Stringenz auf die Hybridisierungsbindung durch Magnetkraft und Temperatur,
f) Bestimmen des Bindungssignals, das in einer an der Oberfläche angebrachten Sandwichstruktur verbleibt,
g) Wiederholen der Schritte e) und f) einmal oder mehrmals und
h) Korrelieren des Bindungssignals, das in einer an die Oberfläche gebundenen Sandwichstruktur verbleibt, mit dem Vorhandensein und/oder der Anzahl von Mutationen, SNP-Varianten oder anderen Varianten in der Zielnukleinsäure;
wobei Magnetfelder mit ansteigenden Spannungen und ansteigenden Temperaturen gleichzeitig oder nacheinander angelegt werden, und wobei die Spannungen und Temperaturen unabhängig voneinander gesteuert werden.

2. Verfahren nach Anspruch 1, wobei das Steuern der Temperatur das Erhöhen der Temperatur der Probe über die Schmelztemperatur der Zielnukleinsäure, die die Mutation enthält, oder der SNP-Variante oder einer anderen Variante umfasst.

3. Verfahren nach einem der vorhergehenden Ansprüche, wobei das Bestimmen des Bindungssignals, das in einer an der Oberfläche angebrachten Sandwichstruktur verbleibt, die optische Detektion der Magnetkügelchen umfasst.

4. Verfahren nach einem der vorhergehenden Ansprüche, wobei mehrere räumlich getrennte Oberflächensonden auf derselben festen Oberfläche und unter denselben Stringenzbedingungen vorhanden sind, wobei jede Sonde eine spezifische Variante der Zielnukleinsäure oder eine spezifische Region des Zielnukleinsäure detektiert, bei der Mutationen oder SNP-Varianten oder andere Varianten vorhanden sein können.

5. Verfahren nach Anspruch 4, wobei die räumlich getrennten Oberflächensonden eine Oberflächensonde für die Wildtypsequenz und eine oder mehrere Oberflächensonden für bestimmte Mutationen oder SNP-Varianten oder andere interessierende Varianten der Zielnukleinsäure umfassen.

6. Verfahren nach Anspruch 4 oder 5, wobei die feste Oberfläche eine Kartusche ist.

7. Verfahren nach einem der vorhergehenden Ansprüche, wobei sich die Zielnukleinsäure vor oder während Schritt d) einem Amplifikationsschritt unterziehet.

8. Verfahren nach einem der vorhergehenden Ansprüche, wobei die Zielnukleinsäure von einem Gen abgeleitet ist, das an der Arzneimittelresistenz beteiligt ist, gegebenenfalls von einem Pathogen, gegebenenfalls von einem viralen Gen oder von einem bakteriellen Gen oder von einem Pilzgen.

9. Verfahren nach Anspruch 8, wobei verschiedene Stämme eines Pathogens durch Detektion von Varianten in der Zielnukleinsäure identifiziert werden.

10. Verfahren nach Anspruch 8 oder 9, wobei das bakterielle Gen ein Gen aus *Mycobacterium tuberculosis* ist.

11. Verfahren nach einem der Ansprüche 1 bis 7, wobei die Zielnukleinsäure von einem Säugetiergen abgeleitet ist, das vorzugsweise menschlich ist, und an Krebs beteiligt ist.

12. Verfahren nach einem der vorhergehenden Ansprüche, wobei das Steuern der Spannung und Temperatur die unspezifische Bindung von Nukleinsäuren mit stark verwandter Sequenz an die Zielnukleinsäure verringert.

## Revendications

1. Procédé de détection de mutations ou de variantes de SNP ou d'autres variantes dans un acide nucléique cible, consistant à:
a) fournir une sonde à billes et une sonde de surface, où chaque sonde est une sonde oligonucléotidique ayant une séquence complémentaire d'une séquence de l'acide nucléique cible, contenant éventuellement un ou plusieurs changements de base par rapport à la séquence de l'acide nucléique cible, et où la sonde à billes est attachée à une bille magnétique, et où la sonde de surface est attachée à une surface solide,
b) fournir un échantillon comprenant l'acide nucléique cible, où l'acide nucléique cible peut contenir une ou plusieurs mutations et/ou variantes par rapport à la séquence de type sauvage de l'acide nucléique cible,
c) mettre en contact l'échantillon avec la sonde à billes et la sonde de surface,
d) permettre à l'acide nucléique cible de s'hybrider avec la sonde à billes et la sonde de surface, en formant une structure en sandwich, en obtenant éventuellement une première lecture du signal de liaison attaché à la surface,
e) appliquer une stringence sur la liaison d'hybridation par la force magnétique et la température,
f) déterminer le signal de liaison restant dans une structure en sandwich attachée à la surface,
g) répéter les étapes e) et f) une ou plusieurs fois, et
h) corréler le signal de liaison restant dans une structure en sandwich attachée à la surface à la présence et/ou au nombre de mutations, de variantes de SNP, ou d'autres variantes dans l'acide nucléique cible,
où des champs magnétiques de tensions croissantes, et de températures croissantes sont appliqués en même temps ou séquentiellement et où lesdites tensions et températures sont commandées indépendamment les unes des autres.

2. Procédé selon la revendication 1, dans lequel le commande de la température comprend l'augmentation de la température de l'échantillon au-dessus de la température de fusion de l'acide nucléique cible contenant la mutation, ou la variante de SNP ou une autre variante.

3. Procédé selon l'une quelconque des revendications précédentes, dans lequel la détermination du signal de liaison restant dans une structure en sandwich attachée à la surface comprend une détection optique des billes magnétiques.

4. Procédé selon l'une quelconque des revendications précédentes, dans lequel plusieurs sondes de surface spatialement séparées sont présentes sur la même surface solide et dans les mêmes conditions de stringence, chacune détectant une variante spécifique de l'acide nucléique cible ou une région spécifique de l'acide nucléique cible où des mutations ou une variante de SNP ou d'autres variantes peuvent être présentes.

5. Procédé selon la revendication 4, dans lequel lesdites sondes de surface spatialement séparées comprennent une sonde de surface pour la séquence de type sauvage et une ou plusieurs sondes de surface pour des mutations particulières ou des variantes de SNP ou d'autres variantes d'intérêt de l'acide nucléique cible.

6. Procédé selon la revendication 4 ou 5, dans lequel la surface solide est une cartouche.

7. Procédé selon l'une quelconque des revendications précédentes, dans lequel l'acide nucléique cible subit une étape d'amplification avant ou pendant l'étape d).

8. Procédé selon l'une quelconque des revendications précédentes, dans lequel l'acide nucléique cible est dérivé d'un gène impliqué dans la résistance aux médicaments, éventuellement d'un pathogène, éventuellement d'un gène viral ou d'un gène bactérien ou d'un gène fongique.

9. Procédé selon la revendication 8, dans lequel différentes souches d'un pathogène sont identifiées par détection de variantes dans l'acide nucléique cible.

10. Procédé selon la revendication 8 ou 9, dans lequel le gène bactérien est un gène de *Mycobacterium tuberculosis.*

11. Procédé selon l'une quelconque des revendications 1 à 7, dans lequel l'acide nucléique cible est dérivé d'un gène mammifère, de préférence humain, impliqué dans le cancer.

12. Procédé selon l'une quelconque des revendications précédentes, dans lequel le commande de la tension et de la température réduisent la liaison non spécifique des acides nucléiques avec une séquence fortement liée à l'acide nucléique cible.
